# EUROPEAN PATENT SPECIFICATION

(11) **EP 2 414 339 B1**
(45) Date of publication and mention of the grant of the patent: **16.01.2013**
(21) Application number: 10714487.5
(22) Date of filing: 05.04.2010
(51) Int. Cl.: C07D 263/22

(54) **KINETIC RESOLUTION OF (4S)-4-PHENYL-3-[5(RS)-(4-FLUOROPHENYL)-5-HYDROXYPENTANOYL]-1,3 OXAZOLIDIN 2-ONE TO (5S) ISOMER VIA LIPASE CATALYZED ENANTIOSELECTIVE ESTERIFICATION OF THE (5R) ISOMER**
KINETISCHE OPTISCHE AUFTRENNUNG VON (4S)-4-PHENYL-3-[5-(RS)-(4-FLUORPHENYL)-5-HYDROXYPENTANOYL]-1,3-OXAZOLIDIN-2-ON ZUM (5S) ISOMER ÜBER DIE LIPASEKATALYSIERTE ENANTIOSELEKTIVE VERESTERUNG DES (5R)-ISOMERS
RÉSOLUTION CINÉTIQUE DE (4S)-4-PHÉNYL-3-[5(RS)-(4-FLUOROPHÉNYL)-5-HYDROXYPENTANOYL]-1,3-OXAZOLIDIN-2-ONE EN ISOMÈRE (5S) PAR ESTÉRIFICATION ÉNANTIOSÉLECTIVE CATALYSÉE PAR UNE LIPASE DE L'ISOMÈRE (5R)

(30) Priority: 02.04.2009 IN KO05772009
(43) Date of publication of application: 08.02.2012
(73) Proprietor: Lupin Ltd., Mumbai, Maharashtra 400 098 (IN)
(72) Inventor: LATHI, Piyush, Suresh, Pune 411 042 (IN); ROY, Bhairab, Nath, Pune 411 042 (IN); SINGH, Girij, Pal, Pune 411 042 (IN); SHRIVASTAVA, Dhananjai, Pune 411 042 (IN)
(74) Representative: Bobbert & Partner
(86) International application number: PCT/IN2010/000224
(87) International publication number: WO 2010/113184

(56) References cited:
- WO-A2-2006/122216
- US-A- 5 618 707
- US-A- 5 849 931
- US-B1- 6 207 822

## Description

### Field of Invention:

The invention relates to novel method for synthesis of optically pure (4S) - 4-phenyl - 3-[(5S)-5-(4-fluorophenyl)-5-hydroxypentanoyl] -1,3 oxazolidin 2-one, of formula I, an intermediate used for the synthesis of ezetimibe (formula II) and (3R, 4S) -4-(3,3'dihydroxybiphenyl-4yl)3-[(3S)-3-(4-fluorophenyl)-3-hydroxypropyl]1 phenylazetidin-2-one (DEPA, formula III) through enzymatic kinetic resolution.

### Background of the Invention:

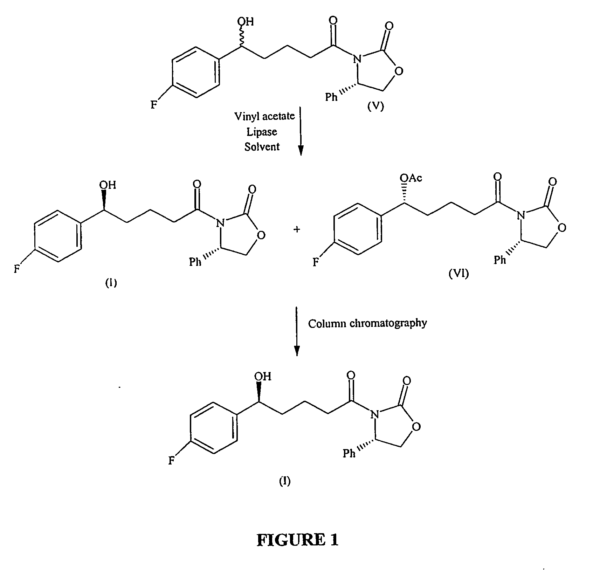

This invention relates to the novel process for the chiral synthesis of ezetimibe and DEPA intermediate of Formula I, (4S) - 4-phenyl - 3- [(5S)-5-(4-fluorophenyl)-5-hydroxypentanoyl] -1,3 oxazolidin 2-one from the corresponding diastereoisomeric alcohols formula V. This is schematically represented in Fig.1.

Ezetimibe (Formula II) (CAS. No. 163222-33-1), 1-(4-fluorophenyl)-3(R)-[3-(4-fluorophenyl) - 3(S) - hydroxypropyl] - 4(S) - (4 - hydroxyphenyl)- 2-azetidinone), a potent and selective cholesterol absorption inhibitor is disclosed in US 5,767,115.

US 7320972 describes DEPA (Formula III) (3R, 4S) -4-(3,3'dihydroxybiphenyl 4yl) 3-[(3S)-3-(4-fluorophenyl)-3-hydroxypropyl] 1-phenylazetidin-2-one as a potent inhibitor of cholesterol absorption.

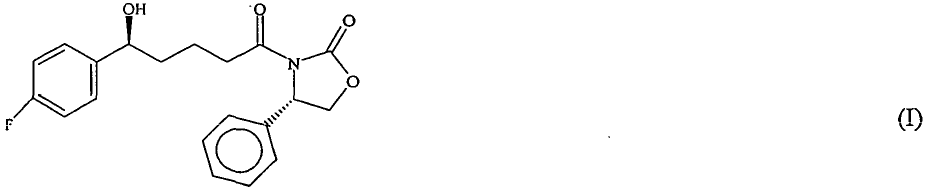

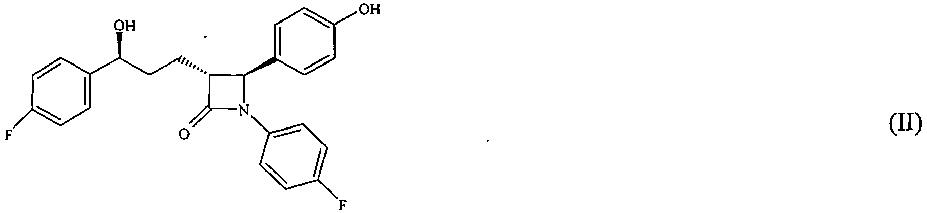

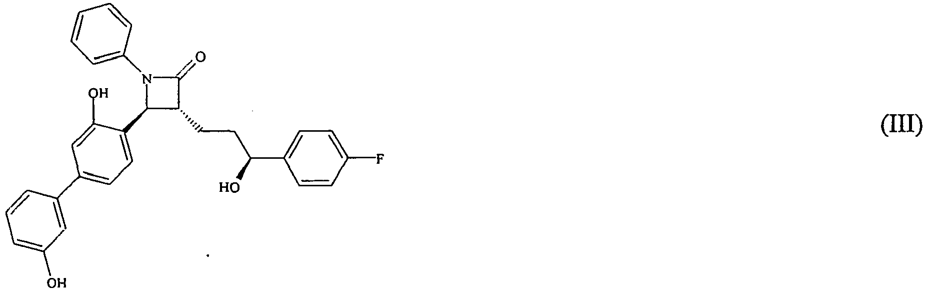

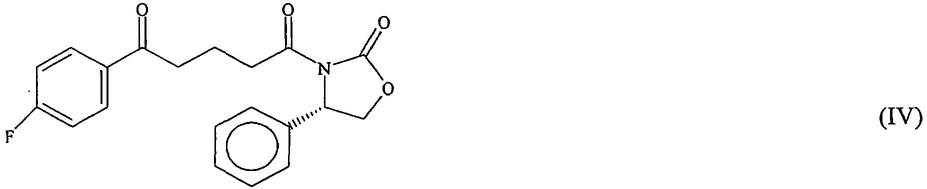

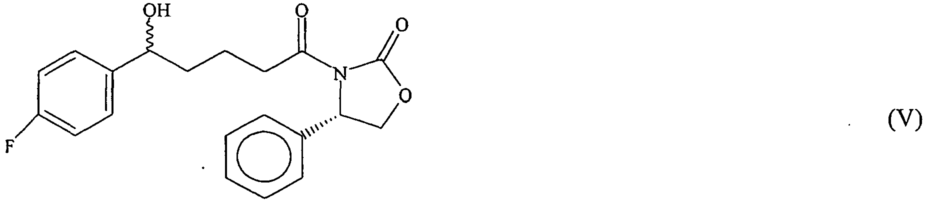

Compound of formula I can be converted to either Ezetimibe (II) or DEPA (III) through the process provided in US 6207822 and WO2006122216 respectively.

Prior art reveals that compound I has been synthesized by following methods:
1) Reduction of corresponding ketone (IV) by using borane as a reducing agent such as Borane-dimethyl sulfide (as in US Pat. No. 6,207,822), Borane-tetrahydrofuran complex (as in Tetrahedron Letters, 44 (2003) 801-804), or Borane diethylaniline complex (as in Tetrahedron Letters, 48(2007) 2123-2125) in presence of chiral catalyst such as (R)-1- methyl-3,3-diphenyltetahydro-3H-pyrrolo [1,2-c][1,3,2] oxazaborole.
2) Compound `I' has also been synthesized by microbial reduction of ketone (IV) as per the process provided in US Pat. No. 5,618,707.

However, most of the reported methods suffer from the following disadvantages
1) Boranes are highly reactive compounds and some are pyrophoric in nature. Most of these are highly poisonous and require special handling precautions, necessitating special operation procedure and higher capital cost.
2) Boranes are volatile and flammable in nature.
3) Some boranes are reported to be unstable on storage, such as borane tetrahydrofuran complex (THF ring opening) and also reported to be explosive on prolonged storage.
4) Borane-dimethyl sulfide has an unpleasant odor and a fact not liked by operation.
5) Enantiomeric purity is never exceed 95%, often requiring further purification.
6) Efficiency of microbial reduction process for desired compound I is low and required high dilution and hence not a practical process. Further, any microbial process is associated with fermentation, which needs special equipment such as sterile condition and environment. Use of autoclave to sterilize the fermentation media, bioreactor, etc.

Non-chiral reduction of corresponding ketone (IV) to racemic hydroxy compounds V (Tetrahedron Letters, 44 (2003)), which is diastereoisomer and in-principle could be separated by crystallization. However, such separation is not simple and easy for the present case due to lower melting points of the individual isomers (39.7°C for compound of formula I).
Summarizing it is evident that there is a need for development of an eco-friendly, hazard free, "green", cost effective process for the synthesis of compound I. This invention provides that.

### Objects of the invention

Thus the object of the present invention is to provide enzymatic kinetic resolutions of 4S-phenyl -3-[(5RS)-5-(4-fluorophenyl)-5-hydroxypentanoyl] -1,3 oxazolidin 2-one to obtain optically pure compound I with enatiomeric purity of at least about 98%.

Other object of the present invention is to provide an eco-friendly and hazard free process for the preparation of compound of formula I.

Another object of the present invention is to provide a process for the preparation of compound of formula I with better efficiency and selectivity.

A further object of the present invention is to provide an improved industrial process for the preparation of compound of formula I that produces minimum by-products.

### Summary of invention

The present invention provides a process for synthesis of 4S-phenyl -3-[(5S)-5-(4-fluorophenyl)-5-hydroxypentanoyl] -1,3 oxazolidin 2-one comprising of resolution of 4S-phenyl -3-[(5RS)-5-(4-fluorophenyl)-5-hydroxypentanoyl] -1,3 oxazolidin 2-one by selective esterification of 4S-phenyl -3-[(5R)-5-(4-fluorophenyl)-5-hydroxypentanoyl] -1,3 oxazolidin 2-one using appropriate esterification reagent in an organic solvent in presence of Lipase enzyme at a temperature ranging from 0° to 100°C, and further isolation.

The esterification agent used in the said process is vinyl acetate

Lipase enzyme used in the process of invention is selected from the group of Lipase AS, Lipase PS, Novozym 435, Lipozyme TL IM, or Lipozyme RM IM; more preferably it is Lipozyme TL IM.

Organic solvent used for the esterification reaction is selected from Toluene, diisopropyl ether or a mixture thereof.

The process of invention is carried out more preferably at 40°C.

The isolation of desired compound I is carried out by column chromatography or crystallization.

### Detailed description of the invention:

The present invention is directed towards the method for preparation of enantiomerically pure 4S-phenyl -3-[(5S)-5-(4-fluorophenyl)-5-hydroxypentanoyl] -1,3 oxazolidin 2-one (I). The method of the present invention involves a kinetic resolution of 4S-phenyl -3-[(SSR)-5-(4-fluorophenyl)-5-hydroxypentanoyl] -1,3 oxazolidin 2-one (VI) by selective acetylation of one isomer in presence of lipase.

Interestingly, the present inventors found that 4S-phenyl -3-[(SR)-5-(4-fluorophenyl)-5-hydroxypentanoyl] -1,3 oxazolidin 2-one (the undesired enantiomer) specifically undergoes the acetylation reaction to give compound of formula VI and the desired compound (I) remains un-reacted. Compound of formula I is easily separated from compound of formula VI by column chromatography or through crystallization by derivatization of the desired alcohol moiety.

Fig 1 shows the inventive process developed for synthesis of optically pure desired compound I.

The process consists of reaction of vinyl acetate with compound of formula V in organic solvent in presence of specified lipase to yield a mixture containing compound I with high yield and high % ee, and undesired isomer is acetylated to give VI. The resulting mixture of alcohol of formula I and acetate of formula VI after usual work-up is purified to individual compounds by column / flash chromatography.

Such enantioselective acetylation reaction of racemic hydroxy compound in presence of lipase is well documented (Hydrolases in organic synthesis, ed Bornscheuer and Kazlauskas, Wiley VCH verlag GmbH & Co, 2006, pp 61-183). However, apriori, it is difficult to predict required enzyme, solvent, temperature and turnover number of catalyst *i.e.* lipase.
The resulting hydroxy compound (Formula I) which is enantiomerically enriched undergoes further reaction to yield the desired essentially enantiomerically pure Ezitimibe (II) and DEPA (III). Compound VI can be reused in enzymatic resolution after subsequent racemization via ester hydrolysis and oxidation to obtain compound IV through known chemical methods, thereby improving overall yield.

The enzyme (or Biocatalyst) may be any protein that will catalyze the enatioselective estrification of one enatiomer to yield the ester of hydroxy compound. Useful enzymes for enantioselectively esterification of hydroxy compound to ester of hydroxy compound may thus include hydrolases, including lipases. Such enzyme may be obtained from a variety of natural sources, including animal organs and microorganisms.

As described hereinafter useful enzymes for the enantioselective conversion of the hydroxy compound to ester of undesired hydroxy compound include lipases obtained from various biological sources (Table 1). Preferably such lipases include enzyme derived from the microorganism Termomyces lanuginosus, such as available from Novozyme A/S.

**Table1: Lipases screening for enantioselective esterification**

| **Sr. No** | **Lipase** | **Enzyme Supplier** |
|---|---|---|
| 1 | Lipase AS "Amano" (*Aspergillus niger)* | Amano Japan |
| 2 | Lipase PS "Amano" (*Burkholderia cepacia*) | Amano, Japan |
| 3 | Novozym 435 (*Candida antarctica* lipase B) | Novozyme A/S |
| 4 | Lipozyme TL IM (*Thermomyces lanuginosus*) | Novozyme A/S |
| 5 | Lipozyme RM IM (*Mucor miehei*) | Novozyme A/S |

The reaction mixture may comprise a single phase or may comprise multiple phases. For example, the enantioselectiove hydrolysis may be carried out in two phases system comprised of solid phase, which contains the enzyme, and an solvent, which contains the initially racemic substrate, the undesired optically active ester and the desired optically active hydroxy compound I.

The amounts of the racemic substrate (Formula V) and the biocatalyst used in the enantioselective hydrolysis will depend on, the properties of the recemic substrate and enzyme. Reaction may generally employ an enzyme loading of about 10% to about 100% and in many cases, may employ an enzyme loading of about 10 to 50% (W/V)

The enantioselective esterification may be carried out over wide range of temperature. For example, the reaction may be carried out at temperature of about 25 °C to a 50 °C, but typically carried out at 40 °C. Such temperatures generally permit substantially full conversion e.g, 95 to 99 % of the one enantiomer in a reasonable period of time e.g. 72 to 120 h without deactivating the enzyme. Enzyme can be reused, and generally the turn-over of immobilized enzyme is high.

The enantioselective esterification may be carried out in different solvents. For example, the reaction may be carried in solvent such as toluene, diisopropyl ether (DIPE), cyclohexane, n-heptane, n-hexane and THF. Preferentially aromatic solvent such as toluene is preferred,

Activated ester used in enantioselective esterification may be consisting of vinyl acetate.

After the completion of reaction, desired hydroxy compound of formula I and ester of undesired enantiomer (VI) is separated out by column chromatography using silica gel as stationary phase and cyclohexane: ethyl acetate (1:1) as a mobile phase.

The present invention is illustrated in more detail by referring to the following Examples, which are not to be construed as limiting the scope of the invention.

Enzymatic screening reactions were performed in an HLC Termomixer. All enzymes used in the screening plate were obtained from commercial enzyme suppliers including Amano (Japan) and Novozyine (Denmark)

### Example 1. Enzyme screening via enzymatic esterification of (R/S) 4-phenyl -3-[(5S)-5-(4-fluorophenyl)-5-hydroxypentanoyl] -1,3 oxazolidin 2-one

Enzyme screening was carried out in HLC parallel thermomixer, which consist of 14 chambers to carry out individual reaction in 10 ml vial (called as individual reactors). Each individual reactor was charged with 3 ml toluene, 100 mg of substrate, and 300mg of vinyl acetate and stirred at room temperature for 15 min. In each reactor different type of lipases (50 % w/w of substrate) was added to initiate reaction. The resulting mixture was stirred at 40 °C for 120 h. reaction was monitor with chiral HPLC for enantioselectivity of Lipases. Retention time of compound I matched with standard sample prepared by known method as provided in US 6,207,822.

### Retention time of compounds on Chiral HPLC

1) Compound I - 20.60 min
2) Compound V -17.44 -and 20.60 min
3) Compound IV-16.07 min

### Chiral HPLC Condition

1) Column: Chiralcel ODH (4.6 X 250 mm).
3) Mobile Phase: n-hexane and ethanol (70:30), flow rate: 0.7 mi/min.; Detection (UV): at 210 nm.

### Compound VI: ¹HNNM (200MH_{z}, CDCl₃):

δ 7.15-7.42 (m, 7H), 7.00(t, 2H), 5.7 (t, 1H), 5.4 (dd, 1H), 4.68 (t 1H), 4.27 (dd 1H), 2.93 (dt 2H), 2.1 (m, 3H), 1.58-1.80 (m, 4H) ppm

**Table2: Lipases screening for enantioselective esterification of Compound V**

| **Lipase** | **% Conversion of one enantiomer (HPLC)** | **% ee_{P}** |
|---|---|---|
| Novozyme 435 | 21 | 51 |
| Lipozyme TL RM | - | - |
| Amano,AS | - | - |
| Amano, PS | 20 | 93 |
| Lipozyme TL IM | 99 | 99 |

### Example 2: Effect of enzyme loading on enzymatic esterification of (R/S) 4-phenyl -3-[(SS)-5-(4-fluorophenyl)-5-hydroxypentanoyl] -1,3 oxazolidin 2-one

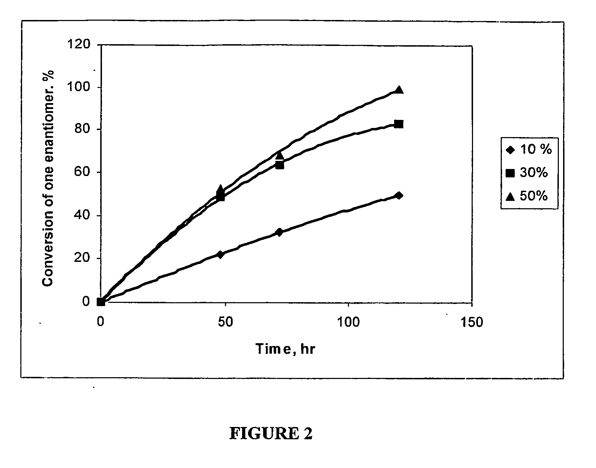

Effect of enzyme loading was carried out in HLC parallel thermomixer, which consist of 14 chambers to carry out individual reaction in 10 ml vial (called as individual reactors). Each individual reactor was charged with 3 ml toluene, 100 mg of substrate, and 300 mg of vinyl acetate and stirred at room temperature for 15 min. In each reactor different % w/w of Lipozyme TL IM lipase was added to initiate reaction. The resulting mixture was stirred at 40 °C for 120 h. reaction was monitor with chiral HPLC for enantioselectivity of Lipases. Fig 2. gives effect of catalysts loading on the rate of reaction.

### Example 3: Effect of different solvent on enantioselective enzymatic esterification of (R/S) 4-phenyl -3-[(5S)-5-(4-fluorophenyl)-5-hydroxypentanoyl] -1,3 oxazolidin 2-one

Effect of enzyme loading was carried out in HLC parallel thermomixer, which consist of 14 chambers to carry out individual reaction in 10 ml vial (called as individual reactors). Each individual reactor was charged with 3 ml of solvent, 100 mg of substrate, and 300mg of vinyl acetate and stirred at room temperature for 15 min. In each reactor of Lipozyme TL IM lipase was added to initiate reaction. The resulting mixture was stirred at 40 °C for 120 h. reaction was monitor with chiral HPLC for enantioselectivity of Lipases. Table 3. Effect of different solvent on enantioselectivity

**Table3: Effect solvent on enantioselective esterification of Formula A**

| **Lipase** | **Solvent** | **% eeₚ** |
|---|---|---|
| Lipozyme TL IM | DIPE | 96 |
| Lipozyme TL IM | Toluene | 99 |

### Example 4. Lipozyme TL IM catalyzed esterification of 4S-phenyl -3-[(5RS)-5-(4-fluorophenyl)-5-hydroxypentanoyl] -1,3 oxazolidin 2-one

4S-phenyl -3-[(5RS)-5-(4-fluorophenyl)-5-hydroxypentanoyl] -1,3 oxazolidin 2-one (1 gm), 3 ml. toluene and 3 g of vinyl acetate were stirred at room temperature for 15 min at an ambient temperature in a HLC thermomixer. The reaction mixture was heated to 40°C and 300 mg of Lipozyme TL IM (Thermomyces lanuginosus) were added to it. Progress of the reaction was monitored using chiral HPLC. After complete conversion of unwanted 4S-phenyl -3-[(5R)-5-(4-fluorophenyl)-5-hydroxypentanoyl] -1,3 oxazolidin 2-one to the Compound VI, reaction was filtered to remove the enzyme. Filtrate was concentrated under vacuum to remove toluene to give crude product, which on column chromatography over silica gel gives 0.37gm (yield 74 %, 99% ee) of compound I, and 0.34 gm (yield 68 %, 99%ee) of compound of formula VI.

## Claims

1. A process for synthesis of 4S-phenyl -3-[(5S)-5-(4-fluorophenyl)-5-hydroxypentanoyl] -1,3 oxazolidin 2-one comprising of resolution of 4S-phenyl -3-[(5RS)-5-(4-fluorophenyl)-5-hydroxypentanoyl] -1,3 oxazolidin 2-one by selective esterification of 4S-phenyl -3-[(5R)-5-(4-fluorophenyl)-5-hydroxypentanoyl] -1,3 oxazolidin 2-one using appropriate esterification reagent in an organic solvent in presence of Lipase enzyme at a temperature ranging from 0° to 100°C, and further isolation.

2. The process as claimed in claim 1 wherein the esterification agent is vinyl acetate

3. The process as claimed in claim 1 wherein the Lipase enzyme is selected from the group of Lipase AS, Lipase PS, Novozym 435, Lipozyme TL IM, or Lipozyme RM IM.

4. The process as claimed in claim 3 wherein the Lipase enzyme is Lipozyme TL IM.

5. The process as claimed in claim 1 wherein the organic solvent is selected from Toluene, diisopropyl ether or a mixture thereof.

6. The process as claimed in claim 1 wherein the esterification reaction is carried out at 40°C.

7. The process as claimed in claim 1 wherein the isolation is carried out by column chromatography or crystallization.

## Patentansprüche

1. Verfahren zur Synthese von 4S-Phenyl-3-[(5S)-5-(4-fluorphenyl)-5-hydroxypentanoyl]-1,3-oxazolidin-2-on, umfassend die optische Auftrennung von 4S-Phenyl-3-[(5RS)-5-(4-fluorphenyl)-5-hydroxypentanoyl]-1,3-oxazolidin-2-on durch selektive Veresterung von 4S-Phenyl-3-[(5R)-5-(4-fluorphenyl)-5-hydroxypentanoyl]-1,3-oxazolidin-2-on unter Verwendung eines geeigneten Veresterungsreagens in einem organischen Lösungsmittel in Anwesenheit von Lipase-Enzym bei einer Temperatur im Bereich von 0 bis 100 °C und weiter die Isolierung.

2. Verfahren nach Anspruch 1, bei dem das Veresterungsmittel Vinylacetat ist.

3. Verfahren nach Anspruch 1, bei dem das Lipase-Enzym aus der Gruppe von Lipase AS, Lipase PS, Novozym 435, Lipozyme TL IM oder Lipozyme RM IM ausgewählt ist.

4. Verfahren nach Anspruch 3, bei dem das Lipase-Enzym Lipozyme TL IM ist.

5. Verfahren nach Anspruch 1, bei dem das organische Lösungsmittel aus Toluol, Diisopropylether oder einer Mischung derselben ausgewählt ist.

6. Verfahren nach Anspruch 1, bei dem die Veresterungsreaktion bei 40 °C durchgeführt wird.

7. Verfahren nach Anspruch 1, bei dem die Isolierung durch Säulenchromatographie oder Kristallisation durchgeführt wird.

## Revendications

1. Procédé de synthèse de 4S-phényle -3-[(5S)-5-(4-fluorophényl)-5-hydroxypentanoyle] -1,3 oxazolidine 2-one comprenant la résolution de 4S-phényle - 3-[(5RS)-5-(4-fluorophényle)-5-hydroxypentanoyle] -1,3 oxazolidine 2-one par estérification sélective de 4S-phényle -3-[(5R)-5-(4-fluorophényle)-5-hydroxypentanoyle] -1,3 oxazolidine 2-one en utilisant un réactif d'estérification approprié dans un solvant organique en présence d'enzyme de Lipase à une température comprise entre 0° et 100°C, pour finir par une isolation.

2. Procédé selon la revendication 1 dans lequel l'agent d'estérification est l'acétate de vinyle.

3. Procédé selon la revendication 1 dans lequel l'enzyme de lipase est sélectionné parmi le groupe constitué de Lipase AS, Lipase PS, Novozyme 435, Lipozyme TL IM ou Lipozyme RM IM.

4. Procédé selon la revendication 3 dans lequel l'enzyme de lipase est le Lipozyme TL IM.

5. Procédé selon la revendication 1 dans lequel le solvant organique est sélectionné parmi le toluène, l'éther de diisopropyle ou un mélange de ceux-ci.

6. Procédé selon la revendication 1 dans lequel la réaction d'estérification est effectuée à 40°C.

7. Procédé selon la revendication 1 dans lequel l'isolation s'effectue par chromatographie à colonne ou cristallisation.
